# EUROPÄISCHE PATENTSCHRIFT

(11) **EP 0 966 516 B1**
(45) Veröffentlichungstag und Bekanntmachung des Hinweises auf die Patenterteilung: **28.05.2003**
(21) Anmeldenummer: 98913624.7
(22) Anmeldetag: 03.03.1998
(51) Int. Cl.: C11D 11/00, C11D 1/66

(54) **VERFAHREN ZUR HERSTELLUNG NEUTRALER ZUCKERTENSIDGRANULATE**
METHOD FOR PRODUCING NEUTRAL SUGAR SURFACTANT GRANULATES
PROCEDE DE PRODUCTION DE GRANULES TENSIOACTIFS DE TYPE SUCRE NEUTRES

(30) Priorität: 12.03.1997 DE 19710153
(43) Veröffentlichungstag der Anmeldung: 29.12.1999
(73) Patentinhaber: Cognis Deutschland GmbH & Co. KG, 40589 Düsseldorf (DE)
(72) Erfinder: LÜDER, Thomas, D-40764 Langenfeld (DE); SCHOLINAKIS, Konstantinos, D-40789 Monheim (DE); GUTSCHE, Bernhard, D-40724 Hilden (DE); HENSEN, Hermann, D-42781 Haan (DE); SEIPEL, Werner, D-40723 Hilden (DE)
(86) Internationale Anmeldenummer: EP9801178
(87) Internationale Veröffentlichungsnummer: WO98040460

(56) Entgegenhaltungen:
- DE-A- 4 209 339
- DE-A- 19 520 105
- DE-C- 19 534 371

## Beschreibung

### Gebiet der Erfindung

Die Erfindung betrifft ein Verfahren zur gleichzeitigen Trocknung und Granulierung von wäßrigen, alkalisch eingestellten Zuckertensidpasten in der dünnen Schicht.

### Stand der Technik

Zuckertenside, wie beispielsweise Alkyloligoglucoside oder Fettsäure-N-alkylglucamide, zeichnen sich durch ausgezeichnete Detergenseigenschaften und hohe ökotoxikologische Verträglichkeit aus. Aus diesem Grund gewinnen diese Klassen nichtionischer Tenside in zunehmendem Maße an Bedeutung. Werden sie bislang in der Regel in flüssigen Formulierungen, wie beispielsweise Geschirrspülmittel oder Haarshampoos eingesetzt, so besteht inzwischen auch ein Marktbedürfnis nach festen, wasserfreien Anbietungsformen, die sich beispielsweise auch in Pulverwaschmittel oder Syndetseifen einarbeiten lassen.

Die Trocknung flüssiger Tensidzubereitungen erfolgt großtechnisch in der Regel durch konventionelle Sprühtrocknung, bei der man die wäßrige Tensidpaste am Kopf eines Turmes in Form feiner Tröpfchen versprüht, denen heiße Trocknungsgase entgegengeführt werden. Diese Technologie ist auf Zuckertensidpasten jedoch nicht ohne weiteres anwendbar, da die für die Trocknung erforderlichen Temperaturen oberhalb der Karamelisierungs- d.h. Zersetzungstemperatur der Zuckertenside liegen. Kurz gesagt: Bei konventioneller Trocknung von Zuckertensidpasten werden verkohlte Produkte erhalten, zudem kommt es zu Anbackungen an der Turmwandung, die in kurzen Abständen eine aufwendige Reinigung erforderlich machen.

In der Vergangenheit hat man versucht, dieses Problem zu umgehen. Aus der Deutschen Patentanmeldung **DE-A1 4102745** (Henkel) ist z.B. ein Verfahren bekannt, bei dem man Fettalkoholpasten eine geringe Menge von 1 bis 5 Gew.-% Alkylglucosiden zusetzt und einer konventionellen Sprühtrocknung unterwirft. Allerdings läßt sich der Prozeß nur in Gegenwart einer großen Menge anorganischer Salze durchführen. In der Deutschen Patentanmeldung **DE-A1 4139551** (Henkel) wird vorgeschlagen, Pasten von Alkylsulfaten und Alkylglucosiden, die jedoch maximal 50 Gew.-% des Zuckertensids enthalten können, in Gegenwart von Mischungen aus Soda und Zeolithen zu versprühen. Hier werden jedoch nur Compounds erhalten, die eine geringe Tensidkonzentration und ein unzureichendes Schüttgewicht aufweisen. Schließlich wird in der Internationalen Patentanmeldung **WO 95/14519** (Henkel) darüber berichtet, Zuckertensidpasten einer Trocknung mit überhitztem Wasserdampf zu unterwerfen; dies ist jedoch technisch sehr aufwendig. Tatsächlich existiert bislang kein zuverlässiges Verfahren, das die Herstellung von qualitativ hochwertigen, praktisch wasserfreien Zuckertensidpulvern bzw. -granulaten gestattet und dabei ohne die Mitverwendung von Trägern während des Trocknungsvorgangs auskommt. Ein weiteres Problem besteht ferner darin, daß Verfahren des Stands der Technik nicht zu den besonders bevorzugten Schwerpulvern mit einem Schüttgewicht oberhalb von 500 g/l mit einem gleichzeitig stark verminderten Staubgehalt führt. Gerade diese beiden Parameter sind jedoch aus wirtschaftlichen, anwendungstechnischen und sicherheitstechnischen Gründen von besonderer Bedeutung.

Aus der deutschen Patentschrift **DE-C1 19534371** (Henkel) ist ein Verfahren zur Herstellung von Zuckertensidgranulaten bekannt, bei dem man wäßrige Pasten von Alkylglucosiden oder Fettsäure-N-alkylglucamiden in der dünnen Schicht trocknet. Die Zubereitungen sind zur antimikrobiellen Stabilisierung stark alkalisch (pH = 11 bis 12) eingestellt. Für kosmetische Produktformulierungen wird jedoch in der Regel ein neutraler bis leicht saurer pH-Wert gewünscht. Die diskontinuierliche Durchführung einer solchen Neutralisation in der Paste nimmt viel Zeit in Anspruch und ist wegen der hohen Pufferwirkung nur begrenzt reproduzierbar, so daß häufiges Nachregeln erforderlich ist. Zudem bewirkt die Unterschreitung eines neutralen pH-Wertes zu einem kräftigen Aufschäumen der Paste unter Freisetzung von Kohlendioxid. Eine anschließende thermische Belastung bei der Trocknung führt ferner zu einem schlecht reproduzierbaren Wiederanstieg des pH-Wertes, so daß die Herstellung eines neutralen Granulats auf diesem Wege nicht ohne weiteres möglich ist.

Demzufolge hat die komplexe Aufgabe der vorliegenden Erfindung darin bestanden, wäßrige alkalisch eingestellte Zuckertensidpasten mit möglichst geringem technischen Aufwand in praktisch wasser- und staubfreie Granulate mit neutralem oder leicht saurem pH-Wert zu überführen, die sich gegenüber den Produkten des Stands der Technik gleichzeitig durch eine verbesserte Farbqualität und Lagerstabilität, hohes Schüttgewicht und gute Rieselfähigkeit auszeichnen sollten.

### Beschreibung der Erfindung

Gegenstand der Erfindung ist ein Verfahren zur Herstellung neutraler Zuckertensidgranulate, bei dem man wäßrige Pasten von
(a) Alkyl- und/oder Alkenyloligoglykosiden und/oder
(b) Fettsäure-N-alkylpolyhydroxyalkylamiden
mit einem Feststoffgehalt von mindestens 20 und vorzugsweise im Bereich von 25 bis 75 Gew.-% in einem horizontal angeordneten Dünnschichtverdampfer bzw. -trockner mit rotierenden Einbauten bis auf einen Restwassergehalt unterhalb von 2, vorzugsweise unter 1,5 und insbesondere unter 1 Gew.-% gleichzeitig trocknet und in stückige Form bringt, welches sich dadurch auszeichnet, daß man die Zuckertensidpasten unmittelbar vor dem Eintritt in den Dünnschichtapparat neutralisiert.

Überraschenderweise wurde gefunden, daß die Eindosierung des Neutralisationsmittels unmittelbar vor dem Eintritt der alkalischen Pasten in den Dünnschichtapparat zu einer raschen und gleichmäßigen Neutralisation führt und Produkte erhalten werden, die sich gegenüber solchen des Stands der Technik als wesentlich hellfarbiger und farbstabiler erweisen. Umgekehrt bedeutet dies, daß man neutralisierte Zuckertensidpasten bei deutlich höheren Temperaturen, d.h. bei erhöhtem Produktdurchsatz trocknen kann, und dennoch Granulate erhält, die in ihrer Farbqualität denen des Stands der Technik nicht nachstehen. Die Produkte weisen eine hohe Schüttdichte im Bereich von 550 bis 650 g/l und einen mittleren Korndurchmesser von 2 bis 4 mm auf, was zu einer Verminderung der unerwünschten Wasseraufnahme und des Zusammenbackens der Partikel führt. Auf diese Weise ist auch eine hohe Lagerstabilität gegeben. Gleichzeitig sind sie staubfrei, d.h. der Anteil an Teilchen mit einem Durchmesser unterhalb von 200 µm liegt unterhalb von 5 Gew.-%, und wesentlich härter als vergleichbare Granulate, was sich vorteilhaft auf die Rieselfähigkeit auswirkt.

### Alkyl- und/oder Alkenyloligoglykoside

Alkyl- und Alkenyloligoglykoside stellen bekannte nichtionische Tenside dar, die der Formel **(I)** folgen,

**R**^{**1**}**O-[G]**_{**p**} **(I)**

in der R¹ für einen Alkyl- undloder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0301298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- undloder Alkenyloligoglykoside sind somit Alkyl- und/oder Alkenyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkylund/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol, Guerbetalkoholen sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide stellen nichtionische Tenside dar, die der Formel **(II)** folgen, 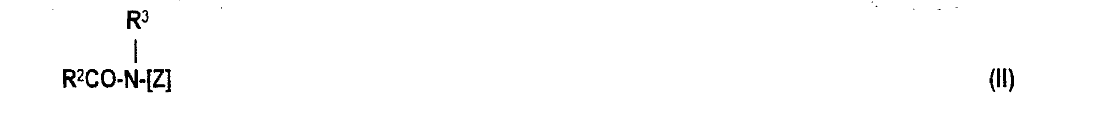 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens. Surf. Det. 25, 8 (1988).** Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(III)** wiedergegeben werden:

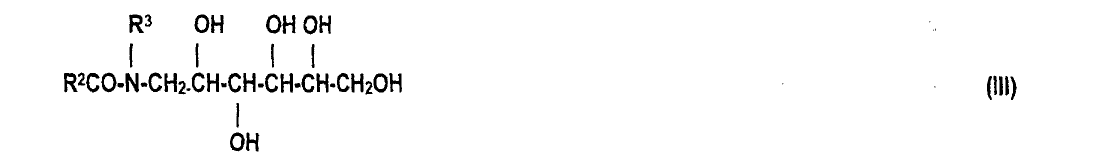

Vorzugsweise werden als Fettsäure-N-alkylpolyhydroxyalkylamide Glucamide der Formel **(III)** eingesetzt, in der R³ für eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel (III), die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Neutralisation

Zur Neutralisation der alkalischen Zuckertensidpasten wird diesen unmittelbar vor dem Eintritt in den Dünnschichtapparat ein kontinuierlicher Strom eines wäßrigen Neutralisationsmittels mit einer solchen Menge und Geschwindigkeit zugegeben, daß sich ein pH-Wert im Bereich von 6,8 bis 7,5 einstellt. Mit Hilfe des schnellaufenden Rotors des Verdampfers wird in sehr kurzer Zeit eine Homogenisierung erreicht, bei der die Bildung von sauren oder alkalischen Nestern zuverlässig vermieden werden kann; auch das unerwünschte Aufschäumen der Mischung findet nicht statt. Die Regulierung des Neutralisationsmittels kann über eine pH-Wert-Messung im Endprodukt erfolgen. Als Neutralisationsmittel kommen grundsätzlich wäßrige Mineralsäuren oder organische Säuren in Frage. Vorzugsweise werden Phosphorsäure, Milchsäure und vorzugsweise Citronensäure in Form wäßriger Lösungen mit einem Feststoffgehalt im Bereich von 25 bis 50 Gew.-% eingesetzt.

### Trocknen und Granulieren in der dünnen Schicht

Die gleichzeitige Trocknung und Granulierung erfolgt in einem horizontal angeordneten Dünnschichtverdampfer bzw. -trockner mit rotierenden Einbauten, wie er z.B. von der Firma VRV unter der Bezeichnung "Flash Dryer" oder von der Firma Vomm unter der Bezeichnung "Turbo Dryer" vertrieben wird. Hierbei handelt es sich, vereinfacht dargestellt, um ein Rohr, das über mehre Zonen hinweg unterschiedlich temperiert werden kann. Über eine oder mehrere Wellen, die mit Blättern oder Flugscharen als rotierende Einbauten versehen sind, wird das pastöse Einsatzmaterial, das über eine Pumpe eindosiert wird, gegen die beheizte Wandung geschleudert, an der die Trocknung in einer dünnen Schicht von typischerweise 1 bis 10 mm Stärke erfolgt. Im Sinne der Erfindung hat es sich als vorteilhaft erwiesen, an den Dünnschichtverdampfer einen Temperaturgradienten von 120 bis 170 (Produkteinlaß) auf 20°C (Produktaustrag) anzulegen. Hierzu können beispielsweise die beiden ersten Zonen des Verdampfers auf 120 bis 190, vorzugsweise 120 bis 130°C geheizt und die letzte auf 20°C gekühlt werden. Höhere Trocknungstemperaturen haben sich im Hinblick auf die thermische Labilität der Einsatzstoffe als nicht vorteilhaft erwiesen. Der Dünnschichtverdampfer wird bei atmosphärischem Druck betrieben und im Gegenstrom mit Luft oder z.B. einem alkalisch eingestellten Gasstrom (Durchsatz bei 0,5 m² Fläche etwa 50 bis 150 m³/h) begast. Die Eintrittstemperatur des Gases liegt in der Regel bei 20 bis 30, die Austrittstemperatur bei 90 bis 110°C,

Die wäßrigen Zuckertensidpasten, die als Einsatzstoffe in Betracht kommen, können einen Feststoffgehalt im Bereich oberhalb von 20, vorzugsweise von 25 bis 75 Gew.-% aufweisen; typischerweise liegt er bei 30 bis 50 Gew.-%. Die Durchsatzmenge ist natürlich von der Größe des Trockners abhängig, liegt beispielsweise für eine Versuchsanlage bei 5 bis 15 kg/h. Es empfiehlt sich, die Pasten bei der Einspeisung auf 40 bis 60°C zu temperieren. In einer bevorzugten Ausführungsform der Erfindung kann man zudem auch mit einer partiellen Produktrückführung arbeiten, d.h. einen Teil des Produktaustrages wieder in die Trocknung zurückführen und mit der wäßrigen Paste vermischen.

Nach der Trocknung hat es sich weiterhin als sehr vorteilhaft erwiesen, die noch etwa 50 bis 70°C heißen Granulate auf ein Förderband, vorzugsweise eine Schwingrinne zu geben und dort rasch, d.h. innerhalb einer Verweilzeit von 20 bis 60 s, mit Umgebungsluft auf Temperaturen von etwa 30 bis 40°C abzukühlen. Zur weiteren Verbesserung der Beständigkeit gegenüber unerwünschter Wasseraufnahme kann man die Granulate auch abpudern, z.B. durch Zugabe von 0,5 bis 2 Gew.-% Kieselsäure, Alkalicarbonat oder dergleichen.

### Gewerbliche Anwendbarkeit

Die nach der erfindungsgemäßen Verfahren erhältlichen Granulaten können entweder im Trockner oder anschließend mit weiteren Inhaltsstoffen von pulverförmigen oberflächenaktiven Mitteln, wie beispielsweise Turmpulvern für Waschmittel abgemischt werden. Es ist ferner problemlos möglich, die Pulver in wäßrige Zubereitungen einzuarbeiten. Tatsächlich werden bei Verwendung der Pulver gegenüber den wäßrigen Ausgangs-pasten keine Unterschiede in den anwendungstechnischen Eigenschaften beobachtet. Auch in Syndet-seifenrezepturen und Zahnpasten lassen sich die Granulate beispielsweise zusammen mit Fettsäuren, Fettsäuresalzen, Stärke, Potyglycol und dergleichen ohne weiteres einarbeiten.

### Beispiel 1

Die Herstellung der Granulate erfolgte in einem Flash Dryer der VRV S.p.A., Mailand/IT. Es handelte sich hierbei um einen horizontal angeordneten Dünnschichttrockner (Länge 1100 mm, Innendurchmesser : 155 mm) mit 4 Wellen und 22 Blättern, deren Abstand zur Wandung 2 mm betrug. Der Trockner besaß drei separate Heiz- bzw. Kühlzonen und eine Wärmeaustauscherfläche von insgesamt 0,44 m². Der Betrieb erfolgte bei Normaldruck. Über eine Schwingpumpe wurde eine auf 50°C temperierte wäßrige Paste eines Kokosalkyloligoglucosids (Plantaren® APG 1200, Feststoffgehalt ca. 50 Gew.-%) mit einem Durchsatz von 11,5 kg/h zusammen mit einer zur Neutralisation ausreichenden Menge einer 30 Gew.-%igen Citronensäurelösung in den Dünnschichtapparat gepumpt, dessen Umfangsheizzonen 1 und 2 auf 185°C und dessen Kühlzone 3 auf 20°C eingestellt worden waren. Die Geschwindigkeit des Rotors betrug 24 m/s. Der Flash Dryer wurde mit Luft (ca. 110 m³/h) begast; die Gasaustrittstemperatur betrug ca. 65°C. Das vorgetrocknete, noch etwa 60°C heiße Granulat wurde auf eine Schwingrinne (Länge 1 m) gegeben, mit Raumluft begast und innerhalb von 30 s auf etwa 40°C abgekühlt. Anschließend wurde es mit etwa 1 Gew.-% Kieselsäure (Sipernat® 50 S) abgepudert. Die Kenndaten des Granulats sind in Tabelle 1 wiedergegeben.

### Beispiel 2

Beispiel 1 wurde wiederholt, die Trocknung in den beiden Zonen 1 und 2 jedoch bei Temperaturen von 125°C durchgeführt.

### Vergleichsbeispiel V1

Beispiel 1 wurde wiederholt, jedoch auf die Neutralisation der Paste verzichtet. Die Ergebnisse sind in Tabelle 1 zusammengefaßt.

**Tabelle 1**

| **Kenndaten des Flash Dryer Granulats (Prozentangaben als Gew.-%]** | | | |
|---|---|---|---|
| **Parameter** | **1** | **2** | **V1** |
| pH-Wert des Produktes | 7,1 | 7,1 | 11,1 |
| Schüttdichte [g/l] | 610 | 612 | 612 |
| Restwassergehalt [%] | 0,2 | 0,2 | 0,2 |
| Produktfarbe [%-Remission] | 75,3 | 81,2 | 67,5 |
| Kornspektrum [%] | | | |
| > 3,15 mm | 38,8 | 38,4 | 39,4 |
| > 1,6 mm | 25,7 | 35,3 | 33,3 |
| > 0,8 mm | 15,2 | 13,4 | 12,4 |
| > 0,4 mm | 9,8 | 8,3 | 7,3 |
| > 0,2 mm | 6,4 | 2,3 | 4,5 |
| > 0,1 mm | 3,3 | 1,6 | 2,3 |
| < 0,1 mm | 0,8 | 0,7 | 0,8 |

## Patentansprüche

1. Verfahren zur Herstellung neutraler Zuckertensidgranulate, bei dem man wäßrige, alkalisch eingestellte Pasten von
(a) Alkyl- und/oder Alkenyloligoglykosiden und/oder
(b) Fettsäure-N-alkylpolyhydroxyalkylamiden
mit einem Feststoffgehalt von mindestens 20 Gew.-% in einem horizontal angeordneten Dünnschichtverdampfer bzw. -trockner mit rotierenden Einbauten bis auf einen Restwassergehalt unterhalb von 2 Gew.-% gleichzeitig trocknet und in stückige Form bringt, **dadurch gekennzeichnet, daß** man die Zuckertensidpasten unmittelbar vor dem Eintritt in den Dünnschichtapparat neutralisiert.

2. Verfahren nach Anspruch 1, **dadurch gekennzeichnet, daß** man Alkyl- und Alkenyloligoglykoside der Formel (I) einsetzt,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Verfahren nach den Ansprüchen 1 und 2, **dadurch gekennzeichnet, daß** man Fettsäure-N-alkylpolyhydroxyalkylamide der Formel (II) einsetzt, 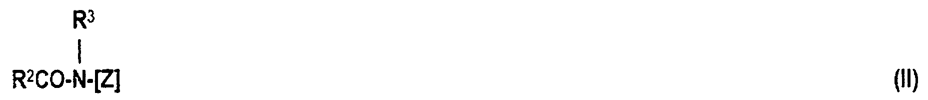 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

4. Verfahren nach den Ansprüchen 1 bis 3, **dadurch gekennzeichnet, daß** man an den Dünnschichtverdampfer bzw. -trockner vom Produkteintrag bis zum -austrag einen Temperaturgradienten von 190 bis auf 20°C anlegt.

5. Verfahren nach den Ansprüchen 1 bis 4, **dadurch gekennzeichnet, daß** man das die noch heißen Granulate nach Ausschleusen aus dem Dünnschichtverdampfer auf einer Schwingrinne mit Umgebungsluft weiter abkühlt.

6. Verfahren nach den Ansprüchen 1 bis 5, **dadurch gekennzeichnet, daß** man die Granulate nach dem Abkühlen abpudert.

## Claims

1. A process for the production of neutral sugar surfactant granules, in which water-containing alkaline pastes of
a) alkyl and/or alkenyl oligoglycosides and/or
b) fatty acid-N-alkyl polyhydroxyalkylamides
with a solids content of at least 20% by weight are dried in a horizontally arranged thin-layer evaporator or dryer with rotating internals to a residual water content below 2% by weight and, at the same time, converted into particulate form, **characterized in that** the sugar surfactant pastes are neutralized immediately before entering the thin-layer evaporator/dryer.

2. A process as claimed as claimed in claim 1, **characterized in that** alkyl and alkenyl oligoglycosides corresponding to formula **(I):**
**R**^{**1**}**O-[G]**_{**p**} **(I)**
in which R¹ is an alkyl and/or alkenyl radical containing 4 to 22 carbon atoms, G is a sugar unit containing 5 or 6 carbon atoms and p is a number of 1 to 10, are used.

3. A process as claimed in claims 1 and 2, **characterized in that** fatty acid-N-alkyl polyhydroxyalkylamides corresponding to formula **(II):** 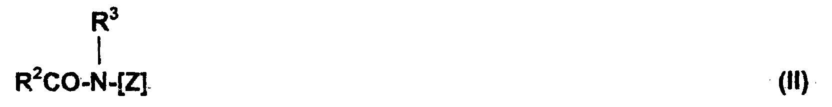 in which R²CO is an aliphatic acyl radical containing 6 to 22 carbon atoms, R³ is hydrogen, an alkyl or hydroxyalkyl radical containing 1 to 4 carbon atoms and [Z] is a linear or branched polyhydroxyalkyl radical containing 3 to 12 carbon atoms and 3 to 10 hydroxyl groups, are used.

4. A process as claimed in claims 1 to 3, **characterized in that** a temperature gradient of 190 to 20°C is applied to the thin-layer evaporator or dryer from the product entrance to the product exit.

5. A process as claimed in claims 1 to 4, **characterized in that,** after leaving the thin-layer evaporator, the still hot granules are further cooled with ambient air on a vibrating chute.

6. A process as claimed in claims 1 to 5, **characterized in that,** after cooling, the granules are dusted with a powder.

## Revendications

1. Procédé de production de granulés d'agents tensioactifs dérivés de sucres neutres dans lequel on sèche des pâles aqueuses ajustées en milieu alcalin de
a) alkyl et/ou alkényloligoglycosides et/ou
b) de N- alkylpolyhydroxyalkylamides d'acide gras
ayant une teneur en matières solides d'au moins 20 % en poids dans un évaporateur ou séchoir en couche mince disposé horizontalement avec des éléments intégrés rotatifs, jusqu'à une teneur en eau résiduelle inférieure à 2 % en poids simultanément, et que l'on amène sous forme fr morceaux,
**caractérisé en ce qu'**
on neutralise les pâtes d'agents tensioactifs dérivés de sucre, immédiatement avant l'entrée dans l'appareillage en couche mince.

2. Procédé selon la revendication 1,
**caractérisé en ce qu'**
on met en oeuvre des alkyl- et des alkényloligoglycosides de formule (I)
**R**^{**1**}**O-[G]**_{**p**} **(I)**
dans laquelle R¹ représente un reste alkyle et/ou alkényle ayant de 4 à 22 atomes de carbone, G représente un reste de sucre ayant 5 ou 6 atomes de carbone et p représente des nombres allant de 1 à 10.

3. Procédé selon les revendications 1 et 2,
**caractérisé en ce qu'**
on met en oeuvre des N- alkylpolyhydroxyalkylamides d'acide gras de formule (II) 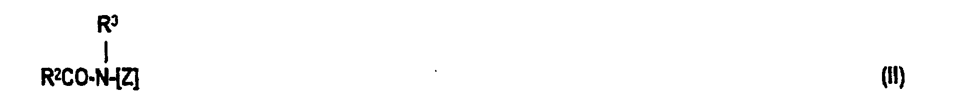 dans laquelle R²CO représente un reste acyle aliphatique ayant de 6 à 22 atomes de carbone, R³ représente de l'hydrogène, un reste alkyle ou hydroxyalkyle ayant de 1 à 4 atomes de carbone et |Z] représente un reste polyhydroxyalkyle linéaire ou ramifié ayant de 3 à 12 atomes de carbone et de 3 à 10 groupes hydroxyle.

4. Procédé selon les revendications 1 à 3,
**caractérisé en ce qu'**
on dispose sur l'évaporateur ou le séchoir en couche mince de l'introduction du produit jusqu'à la décharge du produit un gradient de température allant de 190 à 20°C.

5. Procédé selon les revendications 1 à 4,
**caractérisé en ce qu'**
on refroidit davantage les granulés encore chauds après évacuation de l'évaporateur en couche mince sur un couloir vibrant, avec de l'air ambiant.

6. Procédé selon les revendications 1 à 5,
**caractérisé en ce qu'**
on saupoudre les granulés après le refroidissement.
